# EUROPÄISCHE PATENTSCHRIFT

(11) **EP 0 549 536 B1**
(45) Veröffentlichungstag und Bekanntmachung des Hinweises auf die Patenterteilung: **16.05.2001**
(21) Anmeldenummer: 92811013.9
(22) Anmeldetag: 21.12.1992
(51) Int. Cl.: A61L 2/04

(54) **Sofort-Kleinsterilisationsgerät**
Device for immediate sterilization of small instruments
Appareil pour la stérilisation immédiate de petits instruments

(30) Priorität: 24.12.1991 CH 385891
(43) Veröffentlichungstag der Anmeldung: 30.06.1993
(73) Patentinhaber: K-MIDT GmbH, 1701 Freiburg (CH); Knopf, U.C., Dr., 1701 Freiburg (CH)
(72) Erfinder: Knopf, Ulrich C., Dr., 1701 Freiburg (CH)

(56) Entgegenhaltungen:
- DE-C- 3 941 355
- US-A- 5 068 085

## Beschreibung

Das keimfreie Arbeiten mit Geräten (Skalpellen, Pincetten, Spachteln, Scheren, Zangen und Nadeln) ist Voraussetzung nicht nur für bestimmte Arbeiten in der Human-, Zahn- und Veterinär-Medizin, sondern auch in gewissen Bereichen der Biotechnologie, so in Laboratorien, in denen pflanzliche, oder tierische Gewebe und Zellen in vitro vermehrt, oder an diesen gewisse Eingriffe durchgeführt werden. Zu diesen klassischen Bereichen, in denen die Arbeit mit keimfreien Geräten zur Tagesordnung gehört, kamen in den letzten Jahren andere Bereiche hinzu. Dies infolge der effektiv, oder möglicherweise bestehenden Ansteckungsgefahr insbesondere mit ansteckenden, gefährlichen Krankheitserregern, insbesondere Viren (z.B. dem HIV Virus). Zu diesen neueren Bereichen gehören zum Beispiel kosmetische Arbeiten und Eingriffe, bei denen eine Desinfektion von ArbeitsGeräten nunmehr vermehrt gewünscht wird.
Die Desinfektion von Geräten, wie Skalpellen, Pincetten, Spachteln, Scheren, Zangen und Nadeln (ohne mit dieser Auzählung weitere Geräte auszuschliessen) ist prinzipiell auf verschiedene Art und Weise möglich. Gebräuchlich sind in Spitälern, Arzt-, Zahnarzt- und Tierarztpraxen vor allem die Sterilisation unter Druck (1 atü) und Hitze (110 - 120° C) während 20 - 60 Minuten in sog. Autoklaven, oder aber die Sterilisation in Heissluft-Inkubatoren bei über 200° C, während 3 - 4 Stunden. Diese Verfahren sind anerkanntermassen verlässlich in Bezug auf die Herstellung der Keimfreiheit; sie sind aber, von den Gerätekosten (mehrere Tausend Franken), vom Zeitaufwand (20 Minuten bis mehrere Stunden) und vom Platzbedarf her, relativ aufwendig und deshalb nicht jedermann zugänglich und auch nicht für alle Arbeiten einsetzbar.
Dies ist denn auch der Grund dafür, dass die Sterilisation von Geräten mit den oben erwähnten Autoklaven und Heissluftsterilisatoren bis anhin fast ausschliesslich in gut eingerichteten Spitälern, Praxen und Laboratorien, mit genügend finanziellen Mitteln und Platz, ausgeführt werden konnte.
Besondere Probleme stellt die Sterilisation von Geräten jedoch dort dar, wo infolge Platzmangel (zum Beispiel in relativ kleinen sterilen Arbeitskapellen, sog.
Laminar Flow Cabinets), oder wo infolge eines sich häufig wiederholenden Vorganges, innerhalb von kurzer Zeit immer wieder die gleichen Geräte sterilisiert werden müssen, wie dies zum Beispiel bei der Arbeit mit keimfreien Zell- und Gewebekulturen im biotechnologischen Laboratorium der Fall ist. Bei diesen Arbeiten braucht man heute zur Sterilisation der Arbeits-Geräte meistens noch die offene Flamme (Bunsenbrenner), gefolgt vom Eintauchen der erhitzten Geräte in eine abkühlende, aseptische Flüssigkeit. Dieses Verfahren hat sich in Bezug auf die Keimfreiheit zwar auch bewährt, führt aber einerseits zu unerwünschten Abnützungserscheinungen an den mit der Flamme wiederholt erhitzten Geräten. Andererseits birgt das Verfahren eine erhebliche Unfallgefahr (Brände, Verbrennungen) in sich. Letzteres insbesondere dann, wenn die aspetische Kühl-Flüssigkeit brennbar ist, so wie zum Beispiel der bei solchen Arbeiten häufig verwendete 70%-ige Aethanol.
Andererseits bereitet die Sterilisation von Geräten dort Probleme, wo infolge von fehlenden, finanziellen Mitteln kostspielige Sterilisationsgeräte nicht, oder nicht in genügender Menge angeschafft werden können und wo deshalb als Folge, die Sterilisation ungenügend, oder gar nicht durchgeführt und damit ein erhöhtes Risiko der Uebertragung von Krankheitserregern eingegangen wird.
Die vorliegende Erfindung betrifft ein kostengünstiges, kleines Sterilisations-Gerät, das eine Ganz- oder Teilsterilisation von Geräten wie Pincetten, Klammern, Scheren, Nadeln, Zangen, Rasiermesser, usw. auf kleinem Raum und in sehr kurzer Zeit (10 - 30 Sekunden) erlaubt. Solche Geräte können im Kontakt-Bereich in dem Ansteckungsgefahr herrscht, mit relativ geringem Aufwand und für viele Eingriffe genügend, keimfrei gemacht werden.
Das Sterilisations-Gerät besteht im Wesentlichen aus einem, in kurzer Zeit auf hohe Temperaturen (200 - 300° C) heizbaren Rohrzylinder, der auf seiner Aussenseite durch eine optimale Isolation abgeschirmt wird. Dadurch wird die Wärme vorwiegend ins Innere des Rohres geleitet und es wird so vermieden, dass sich andere Geräteteile, zum Beispiel elektronische und Elektrizität leitende Teile, aber auch das Gehäuse, über 50° C erhitzen. Dadurch wird vermieden, dass Geräteteile die eine Temperatur von über 60° C nicht ertragen nicht beschädigt und die das Gerät umgebenden Teile, oder Abstellflächen und die Geräte- Benutzer durch Ueberhitzung (Brandgefahr) und Verbrennungen nicht gefährdet werden. Das Metallrohr wird mit einem inerten, hochtemperaturbeständigen, wärmeleitenden Material (zum Beispiel Sand, Glaskugeln, usw. ) gefüllt, in welches nach dessen Erhitzung auf ca. 250° C die zu sterilisierenden Geräte für kurze Zeit (10 - 30 Sekunden) eingesteckt werden, so dass auch sie im Kontaktbereich sofort auf die vorbestimmte, hohe Temperatur von 250° C gebracht werden. Dadurch werden die an den Kontaktflächen vorhandenen Mikroorganismen (Viren, Bakterien, mikroskopische Pilze, usw.) innerhalb kürzester Zeit (Sekunden) abgetötet und es wird damit erwiesenermassen ein guter Sterilisationseffekt erzielt.

Der Aufbau des Gerätes ist aus den Figuren 1 - 3 ersichtlich, ohne dass dabei die Ausführung der Erfindung in irgend einer Weise eingeschränkt werden soll. Fig. 1 zeigt eine Aussenansicht des Gerätes, Fig. 2 zeigt einen Querschnitt durch das Gerät und Fig. 3 zeigt einen Querschnitt durch den Sterilisationsgefäss-Teil, nachdem dieser aus dem Gehäuse entfernt wurde.

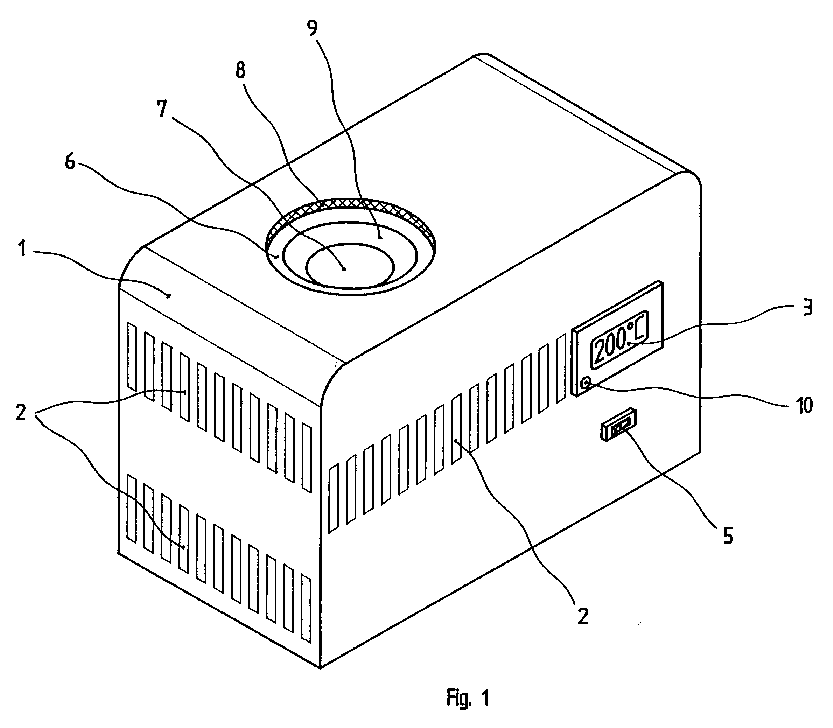

Fig. 1 zeigt eine Aussenansicht einer Ausführung eines Sterilisations-Gerätes. Sichtbar ist das Metallgehäuse (1), das mit Kühlschlitzen (2) durchzogen ist. Sichtbar sind ferner die Temperaturanzeige (3) die auf dem Mikroprozessor angebracht ist, ein Netzschalter (5) und eine Oeffnung im Gehäuse (6) mit der der Benützer Zugang zum Sterilisationsgefäss (7) erhält. Sichtbar ist dabei auch das Isolationsrohr (8) und die das Isolationsrohr in Position haltende Metallmanschette (9). Der Oberteil des Gehäuses (1) drückt leicht auf das Isolationsrohr (8) und hält dieses auch so in der gewünschten Position. Mit Hilfe einer Drucktaste (10) kann die Soll-Wert Temperatur am Mikroprozessor verändert bzw. eingestellt werden.

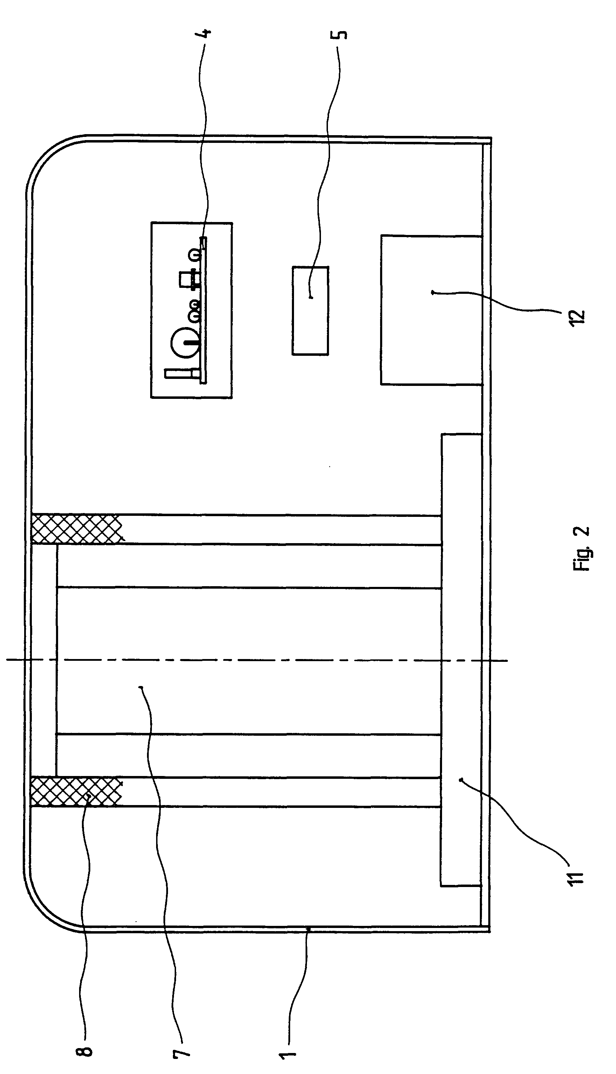

Fig. 2 zeigt einen Querschnitt durch das Sterilisations-Gerät, nämlich das Gehäuse (1), das Isolationsrohr (8) das vom Gehäuse-Oberteil nach unten gedrückt, und so in Position gehalten wird, das Sterilisationsgefäss (7), die plane Boden-Isolationsplatte (11), den für den Betrieb des Mikroprozessors notwendigen Transformator (12), den Netzschalter (5), sowie den Mikroprozessor (4), der nach Festsetzen einer Soll-Temperatur die Stromzufuhr zur Heizmanschette einschaltet, oder unterbricht und damit die Innen-Temperatur des Sterilisationsgefässes reguliert.

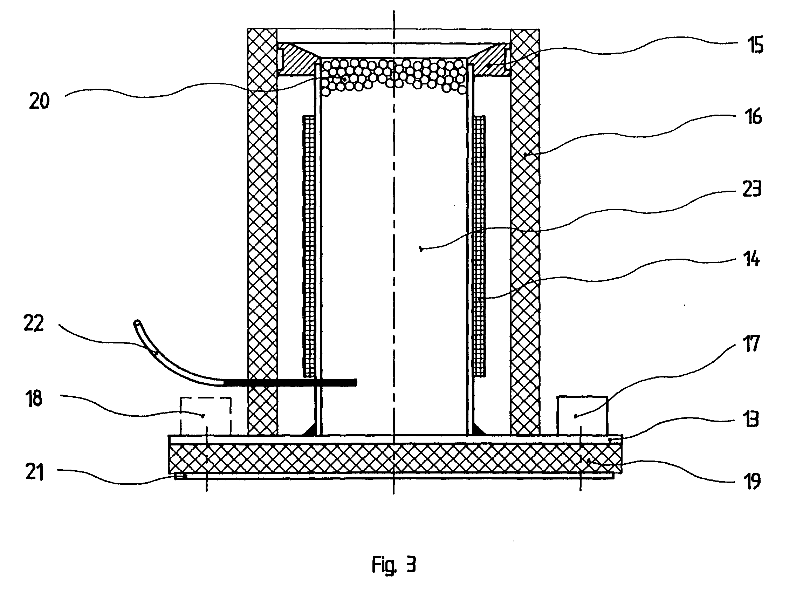

Fig. 3 zeigt den aus dem Gehäuse entfernte Sterilisationsgefäss-Teil. Es besteht aus einem Metallrohr (23), das unten fest mit einer Metallplatte (13) verbunden ist. In das Metallrohr wird vor dem Erhitzen das inerte Material, zum Beispiel Glaskugeln (20) eingefüllt. Das Metallrohr wird von einer Heizmanschette (14) umschlossen. An dem oberen Ende wird die reversibel entfernbare Metallmanschette (15) aufgepresst, die einerseits verhindert, dass Materialien (zum Beispiel Glaskugeln) in den Bereich der Heizmanschette eindringen, andererseits das Isolationsrohr (16) in dem gewünschten, gleichmässigen Abstand zum Metallrohr (23) hält und schliesslich als Wärmebarriere gegen oben dient. In das Metallrohr (23) und das inerte Material (20) dringt eine Temperatursonde (22), die mit dem Mikroprozessor (4) verbunden ist und dort die Ist- Temperatur misst. Auf der Metallplatte (13) ist eine Temperatursicherung (17) angebracht, die verhindern soll, dass das Gerät bei Ausfall der elektronischen Temperaturregelung unkontrolliert weiterheizt. Auf der Metallplatte (13) kann zudem ein weiterer Bimetall Thermostat (18) angebracht werden, nämlich dann wenn die aufwendigere elektronische Temperatur-Regelung - durch eine kostengünstigere Bimetall Thermostaten Regelung ersetzt wird. Die Metallplatte (13) wird gegen unten durch die Isolationsplatte (19) abgeschirmt und letztere, wiederum von unten, durch ein Metallblech (21) gestützt. Die Metallplatte (13) inkl. Metallrohr, Isolationsplatte (19) und Metallblech (21) sind miteinander fest verbunden.

Damit ein gemäss Fig. 1 - 3 3 konstruiertes Gerät entsprechend seinem Zweck gebraucht werden kann, wird es am Arbeitsplatz an eine Stromquelle angeschlossen. Der Mikroprozessor (4) wird so eingestellt, dass sich die im Metallrohr (23) eingefüllten Glaskugeln (20) auf eine hohe Temperatur (um 250° C) erhitzen. Dies geschieht innerhalb von wenigen Minuten. Anschliessend können die zu sterilisierenden Geräte wie oben beschrieben während kurzer Zeit (10 - 30 Sekunden) in die heissen Glaskugeln (20) gesteckt werden, wobei sie an der Kontaktstelle zu den heissen Glaskugeln innerhalb dieser kurzen Zeit keimfrei gemacht werden.
Die so sterilisierten Geräte können anschliessend entweder sofort gebraucht, oder aber ohne Gefahr in eine aseptische, kühlende Flüssigkeit eingetaucht und so wenn angezeigt, vor dem Gebrauch noch abgekühlt werden. Nach erfolgtem Eingriff, können die Geräte in kürzester Zeit und auf die gleiche Weise wieder sterilisiert werden. Auf diese Weise kann ein und dasselbe Instrument, zum Beispiel eine in der Transplantation von in vitro kultiviertem Gewebe verwendete Pincette, direkt am Arbeitsplatz (Sterilkapelle), viele Male pro Tag, innert kürzester Frist, keimfrei gemacht und repetitiv wieder eingesetzt werden.

Die Schwierigkeit in der Konstruktion eines solchen Gerätes liegt darin, dass das Gerät ermöglichen muss, das Metallrohr auf relativ hohe Temperaturen (220 - 300° C) zu erhitzen und dieses in einem raumsparenden (kleinen) Gehäuse unterzubringen, ohne dass dadurch die notwendigen, übrigen Geräteteile, insbesondere die elektronischen Komponenten und elektrischen Kabel, oder das Gehäuse so stark erhitzt werden, als dass dies für diese zu einer Beschädigung und/oder den Benützer des Gerätes in Gefahr (Verbrennung, Brand) bringen könnte. Die Lösung des Problems bedingte, dass die Materialien so gewählt und fabriziert werden und die Konstruktion des Gerätes so erfolgen mussten, dass auch bei maximaler Metallrohrtemperatur (ca. 280° C), sowohl das Gehäuse als auch das Gehäuseinnere nicht mehr als 50° C erhitzt werden dürfen. Dieses Problem haben wir einerseits durch die Art der Konstruktion des Sterilisationsgefässes, insbesondere auch der Metallmanschette (17), der Wahl der Isolations-Materialien (Aluminium Silikat mit, oder ohne Eisen- und Titanoxid), der Konstruktion und der Form des Isolationsrohres (18) und schliesslich der Konstruktion des Gehäuses (1), gelöst. Dem Isolationsrohr (18) wird vor seiner Montag durch Druck, Hitze und unter Verwendung eines Bindemittels die gewünschte Form eines Zylinderrohres gegeben. Das Isolationsrohr umgibt das Metallrohr mit der Heizmanschette und wird durch den Oberteil des Gehäuses leicht nach unten auf die Metall- und Isolationsplatte gedrückt. Durch den leichten Druck des Gehäusedeckels kann ein Hohlraum zwischen Sterilisationsgefäss und Gehäuse vermieden werden, was zur Folge hat, dass einerseits kein inertes Material (zum Beispiel Glaskugeln) ins Innere des Gehäuses dringen kann. Dies muss vermieden werden, weil das inerte Material von Zeit zu Zeit gereinigt, oder ausgewechselt werden muss. Andererseits wird dadurch ein Teil der Wärme vom Isolationsrohr auf das Gehäuse übertragen und so, statt ins Innere des Gerätes, nach aussen abgestrahlt . Das besonders wärmedämmende starre Isolationsmaterial (Aluminium Silikat mit, oder ohne Eisen und Titanoxid) wird auch in planer Form verwendet um das auf einer festen Metallplatte angebrachte Metallrohr gegen den Gehäuseboden zu isolieren. Dabei wird das Isolationsmaterial zwischen die Metallplatte (13) und ein Metallblech (21) gepresst. Die ganze Konstruktion kann sodann direkt auf den Gehäuseboden (Gehäuse-Unterteil) des Gerätes geschraubt werden. Dadurch wird es möglich, ein kleines, und zugleich optimal isoliertes Gerät zu konstruieren.
Die Metallplatte (13) dient sowohl als Abschluss (Boden) des Metallrohres (23), als auch als Vorrichtung um eine auswechselbare Bimetall-Temperatursicherung (17), oder einen auswechselbaren Bimetallthermostaten (18) anzubringen. So funktioniert die mit dem heizbaren Rohr verbundenen Metallplatte nicht nur als mechanische Befestigungseinheit, sondern gleichzeitig auch als wärmeleitendes Medium. Diese Konstruktion bewirkt, dass die Dimensionen des Gerätes erneut minimal gehalten und zudem die Konstruktionskosten vermindert werden können.
Sie erlaubt zudem, dass die Bimetallthermostaten, oder Bimetallsicherungen, die nur eine beschränkte Lebensdauer haben, wenn nötig mit sehr geringem Aufwand ausgewechselt werden können.
Die Verwendung eines Mikroprozessors (4) zusammen mit einer ins Metallrohr führenden Sonde (22), erlaubt eine rasche, exakte, kontinuierliche, stromsparende Regulation der Temperatur des im Metallrohr (23) erhitzten , inerten Materials (20).
Der Ersatz der elektronischen Temperaturregulation mit dem Mikroprozessor (4) durch einen kleineren Bimetallthermostaten (18) könnte deshalb nur dort erfolgen, wo eine rasche, präzise Regelung und Kontrolle der Temperatur nicht erforderlich sind. Zwar ist eine solche Version mit Sicherheit kostengünstiger, weil dabbei nebst dem Mikroprozessor (4) und dem dafür benötigten Transformator (12), auch die elektronische Temperaturanzeige (3) und die Temperatursonde (22) wegfallen können; doch kommt sie sowohl von der Sicherheit in Bezug auf Funktionstüchtigkeit (Keimfreiheit der Geräte), wie auch von der Langlebigkeit des Gerätes (Bimetallthermostate haben eine beschränkte Lebensdauer) nicht an die elektronische Lösung heran.

## Patentansprüche

1. Vorrichtung zum Heisssterilisieren und zum Inkubieren von Geräten für das biologische und medizinische Laboratorium, bestehend aus einem elektrisch beheizten und mit Thermostaten versehenen, nach oben offenen, vorzugsweise zylindrischen Metallgefäss (23), dadurch gekennzeichnet, dass das Metallgefäss an seinem oberen Ende eine reversibel angebrachte ringförmige Metallmanschette (15) mit einer dem inneren Durchmesser des Rohrs entsprechenden Ringöffnung trägt, wobei diese Oeffnung nach oben kegelförmig auf den Innendurchmesser des Rohrs übersteigenden Durchmesser aufgeweitet ist und der Aussendurchmesser der gewünschten Distanz des Metallgefässes zur Isolation beträgt und das Metallgefäss mit Partikeln aus einem inerten, wärmeleitenden Material gefüllt ist.

2. Vorrichtung gemäss Anspruch 1, dadurch gekennzeichnet dass das Metallgefäss (23) mit Partikeln (20) aus einem inerten, wärmeleitenden Material gefüllt ist, in welche die zu inkubierenden und zu sterilisierenden Geräte für ein kurze Zeit eingesteckt und dadurch auf eine vorbestimmte Temperatur gebracht werden können.

3. Vorrichtung gemäss Ansprüchen 1 und 2, dadurch gekennzeichnet, dass das Metallgefäss (23) ein zylindrisches Rohr aus einem wärmeleitenden Metall, vorzugsweise Chromstahl, mit einer daran angeschweissten, planen Bodenplatte (13) ist, wobei die Bodenplatte seitlich über den Umfang des Rohres herausragt und mit Mitteln zur festen Verbindung mit einem Gehäuse versehen ist.

4. Vorrichtung gemäss einem der Ansprüche 1 bis 3 dadurch gekennzeichnet, dass das zylindrische Metallrohr von einer Widerstandsdrähte enthaltenden elektrischen Heizmanschette (14) umschlossen ist.

5. Vorrichtung gemäss den Ansprüchen 1 bis 4, dadurch gekennzeichnet, dass das Metallrohr (23), die Heizmanschette (14) und die ringförmige Metallmanschette (15) von einem zylindrischen, starren Rohr (16) aus isolierendem Material umgeben ist, welches seitlich von der ringförmigen Metallmanschette (15) und von oben vom Gehäuseoberteil (1) in gleichmässiger Distanz zum Metallrohr (23) gehalten wird, so dass die von der Heizmanschette (14) erzeugte Wärme vorwiegend ins Metallrohr (23) und auf das darin befindliche inerte Material (20) und nicht auf die elektronischen Teile (4, 12, 5) im Innern des Apparates oder auf das Gehäuse (1) abstrahlt.

6. Vorrichtung gemäss Anspruch 3, dadurch gekennzeichnet dass die Bodenplatte (13) auf ihrem das Rohr überragenden Teil einen oder mehrere Thermostate (17) aus Bimetall trägt, welche die Maximaltemperatur der Vorrichtung durch Unterbrechung des Heizstromes begrenzen.

7. Vorrichtung gemäss Anspruch 3, dadurch gekennzeichnet, dass zwischen die Bodenplatte (13) und dem Gehäuse (1) eine Isolationsplatte (19) angebracht wird, die erlaubt die Bodenplatte (13) fest mit dem Gehäuseteil zu verbinden und zwar so dass die von der Heizmanschette (14) auf die Bodenplatte übertragene Wärme vorwiegend ins zylindrische Rohr (23) und nicht auf die elektronischen (4, 5, 12) und Elektrizität leitenden Teile des Gerätes oder auf das Gehäuse (1) des Gerätes übertragen wird.

8. Vorrichtung gemäss einem der Ansprüche 1 bis 7, dadurch gekennzeichnet, dass das Isolationsrohr (16) und die Isolationsplatte (19) aus mit einem Bindemittel verfestigten, starren, Aluminium-Silikatfasern besteht.

9. Vorrichtung gemäss einem der Ansprüche 1 bis 7
dadurch gekennzeichnet, dass das Isolationsrohr (16) und die Isolationsplatte (19) aus gepresstem und mit einer Folie abgeschlossenen und geformten starren Material hauptsächlich aus Siliziumoxid, Eisenoxid, Titanoxid und Aluminiumoxid besteht.

10. Vorrichtung gemäss einem der Ansprüche 1 bis 7, dadurch gekennzeichnet, dass die Partikel aus inertem Material (20) Kugeln aus Glas mit einem Durchmesser von höchstens 3 mm sind.

11. Vorrichtung gemäss einem der Ansprüche 1 bis 7, dadurch gekennzeichnet, dass die Partikel aus inertem Material (20) Sandkörner sind.

12. Vorrichtung gemäss einem der Ansprüche 1 bis 7, dadurch gekennzeichnet, dass die Partikel aus inertem Material (20) Graphitkörner sind.

13. Vorrichtung gemäss einem der Ansprüche 1 bis 12, dadurch gekennzeichnet, dass die elektrische Heizvorrichtung (14) des Metallrohrs mit einer Temperatursonde (22) ausgestattet ist, die die Temperatur der Partikel im Innern des Rohres misst und die Temperatur der Partikelfüllung durch einen elektronischen Mikroprozessor (4) derart gesteuert wird, dass sie auf einen bestimmten , voreinstellbaren Wert gebracht und dort konstant gehalten wird.

14. Vorrichtung gemäss Anspruch 13, dadurch gekennzeichnet, dass der elektronische Mikroprozessor (4) mit einer Vorrichtung zur Einstellung der Arbeitstemperatur, sowie mit einer von aussen ablesbaren Temperaturanzeige versehen ist.

15. Vorrichtung gemäss einem der Ansprüche 1 bis 14, dadurch gekennzeichnet, dass das heizbare Metallrohr (23) mit der Partikelfüllung und dem an der Grundplatte angebrachten Sicherheitsthermostaten (17), der Mikroprozessor (4) für die Steuerung und die von aussen ablesbare Anzeige, sowie die von aussen bedienbare Temperatureinstellvorrichtung in einem möglichst kleinen, gemeinsamen Gehäuse (1) untergebracht sind, welches am oberen Zugang eine kreisförmige Oeffnung (7) aufweist, wobei die Abmessungen des Gehäuses und die darin angebrachten Kühlschlitze (2), sowie das das Metallrohr umgebende Isolationsrohr (16) und die Bodenplatte abgrenzende Isolationsplatte (19) so angeordnet sind, dass die elektronischen Teile und die Gehäuseteile auch bei Metallrohrtemperaturen von über 200° C nie eine solche von 50° C übersteigende Temperatur annehmen.

## Claims

1. Apparatus for hot sterilization and incubation of instruments for the biological and medical laboratory, consisting of an electrically heated and with thermostats administered, preferably cylindrical, metal vessel (23) opened on the top, characterized by the feature that the metal vessel has on the top end a reversibly fixed metal cuff (15), with an opening corresponding to the inside diameter of the cylindrical tube, whereas the opening is being enlarged as a cone from the inside to the outside and where the outside diameter corresponds to the desired distance of the cylindrical metal tube to the insulation and that the metal tube is filled with particles made of an inert, heat conducting material.

2. Apparatus according to claim 1, characterized by the fact, that the metal vessel (23) is filled with particles (20) made of an inert, heat conducting material in which the instruments to be incubated and to be sterilized are put for a short period of time and thus be brought to a predetermined temperature.

3. Apparatus according to claims 1 and 2, characterized by the fact , that the metal vessel (23) is a cylindrical tube made of a heat conducting metal, preferably chrome steel, with a flat base plate (13) welded to it, whereas the base plate exceeds the diameter of the metal tube and has means to be fixed firmely with a casing.

4. Apparatus according to one of the claims 1 to 3, characterized by the fact that the cylindrical metal tube is being surrounded by an electrical heating cuff (14) containing resistance wires.

5. Apparatus according to claims 1 to 4, characterized by the feature that the metal tube (23), the heating cuff (14) and the circular metal cuff (15) are surrounded by a cylindrical, stiff tube (16) made of insulating material, which is being kept laterally by the circular metal cuff (15) and from the top by the upper part of the casing (1), in equal distance to the metal tube (23) so that the heat generated by the heating cuff (14) is being transmitted in to the metal tube (23) and on the inert material inside (20) the tube and not on the electronical parts (4, 12, 5) inside the apparatus or the casing (1).

6. Apparatus according to claim 3, characterized by the feature that the flat base plate (13) has fixed on its part exceeding the metal tube one or several thermostats (17) made of bimetall which limit the maximum temperature of the apparatus by interruption of the electrical heating current.

7. Apparatus according to claim 3, characterized by the feature that between the base plate (13) and the casing, an insulating plate (19) has been installed which allows to attach the base plate (13) firmely on to the casing so that the heat transmitted from the heating cuff will be transmitted principally into the cylindrical tube (23) and not on to the electronic (4, 5, 12) and electricity conducting parts or on the casing (1) of the apparatus.

8. Apparatus according to one of the claims 1 to 7 characterized by the feature that the insulating tube (16) and the insulating plate (19) consist of stiff, fibres of aluminium silicat, solidified by a binding agent.

9. Apparatus according to one of the claims 1 to 7 characterized by the feature that the insulating tube (16) and the insulating plate (19) are made out of a pressed, formed, stiff material made of siliciumoxid, ironoxide, titaniumoxid and aluminiumoxid enclosed by a foil.

10. Apparatus according to one of the claims 1 to 7, charcterized by the feature that the particles of inert material (20) are glass beads of a diameter of maximum 3 mm.

11. Apparatus according to one of the claims 1 to 7, characterized by the feature that the particles of inert material (20) consists of sand grains.

12. Apparatus according to claims 1 to 7, characterized by the feature that the particles of inert material (20) are graphite-grains.

13. Apparatus according to claims 1 to 12 characterized by the feature that the r the electrical heating installation (14) of the metal tube contains a temperature probe (22) measuring the temperature of the particles inside the tube allowing the temperature of the particle filling to be controlled by a microprocessor (4), in order to heat it to a determined, preset value and to hold it constant.

14. Apparatus according to claim 13, characterized by the fact that the electronic microprocessor (4) has means for setting the working temperature and a temperature indication which can be read from the outside.

15. Apparatus according to one of the claims 1 to 14, characterized by the fact, that the heated metal tube (23) and the particle filling (14) and the safety thermostat (17) fixed on the base plate, the microprocessor for the regulation and the outside setting for the temperature are placed in a common casing (1), which is as small as possible and has a circular opening (7) on the top where the dimensions of the casing and cooling-spaces (2), the metal tube circumvented by the insulating tube (16) and the insulating plate (19) separating the base plate are positioned so that the electronical parts and the parts of the casing will never exceed 50°C even so the metal tube will have a temperature of over 200° C.

## Revendications

1. Appareil de stérilisation à chaud et d'incubation pour des instruments pour les laboratoires de biologie et de médecine composé d'un récipient (23) cylindrique en métal avec une ouverture en haut, chauffé électriquement et muni de thermostats, caractérisé par le fait que le récipient en métal porte autour de son extrémité supérieure un anneau (15) métallique amovible dont l'ouverture correspond au diamètre intérieur du tube cylindrique et que cette ouverture s'élargit de façon conique de l'intérieur à l'extérieur de sorte à ce que le diamètre extérieur correspond à la distance voulue du récipient cylindrique en métal jusqu'à l'isolation et que le tube métallique est rempli de particules fait d'un matériel inerte conduisant la chaleur.

2. Appareil conforme à la revendication 1, caractérisé par le fait que le récipient en métal (23) est rempli de particules (20) fait d'un matériel inerte conduisant la chaleur, dans lequel les instruments à incuber et à stériliser sont plongés pour une courte période de temps afin d'être chauffés à une température prédéterminée.

3. Appareil conforme aux revendications 1 et 2 caractérisé par le fait, que le récipient en métal (23) est un tube cylindrique fabriqué en métal conduisant la chaleur, de préférence en acier inox, avec une plaque de base plane (13) soudé au tube de façon à ce que cette plaque de base plane dépasse latéralement le diamètre du tube et qu'elle a des moyens permettant de la fixer fermement à un boîtier.

4. Appareil conforme à une des revendications 1 à 3 caractérisé par le fait que le tube cylindrique en métal est entouré d'un manchon (14) électrique chauffant contenant des files de résistance.

5. Appareil conforme aux revendications 1 à 4 caractérisé par le fait que le tube en métal (23), le manchon chauffant (14) et la manchette métallique circulaire (15) sont entourés par un tube cylindrique, rigide (16), fait d'un matériel isolant, tenu latéralement à distance égale du tube métallique (23) par la manchette annulaire métallique (15) et d'en haut par la partie supérieure du boîtier (1) afin que la chaleur produite par le manchon chauffant (14) se transfère avant tout vers intérieur du tube métallique (23) et le matériel inerte (20) qui se trouve à l'intérieur et non pas aux parties électroniques (4, 12, 5) et au boîtier (1).

6. Appareil conforme à la revendication 3 caractérisé par le fait qu'un ou plusieurs thermostats (17) bimétalliques sont fixés sur la partie de la plaque de base (13) dépassant le diamètre du tube, permettant de limiter la température maximale de l'appareil en coupant le courant électrique du chauffage.

7. Appareil conforme à la revendication 3 caractérisé par le fait qu'une plaque isolante (19) est placée entre la plaque de base (13) et le boîtier (1), afin de permettre de fixer la plaque de base (13) fermement au boîtier de manière à ce que la chaleur qui se transmet du manchon électrique (14) sur la plaque de base (13) va surtout à intérieur du tube cylindrique (23) et non pas aux parties électroniques (4, 5, 12) et électriques ou sur le boîtier (1) de l'appareil.

8. Appareil conforme à une des revendications 1 à 7 caractérisé par le fait que le tube isolant (16) et la plaque d'isolation (19) sont fabriqués de fibres rigides en silicate d'aluminium endurcies à l'aide d'une colle.

9. Appareil conforme à une des revendications 1 à 7 caractérisé par le fait que le tube d'isolation (16) et la plaque d'isolation (19) sont faits d'un matériel rigide, pressé, formé et enveloppé dans une feuille consistant principalement d'oxyde de silicium, d'oxyde de fer, d'oxyde de titanium et d'oxyde d'aluminium.

10. Appareil conforme à une des revendications 1 à 7 caractérisé par le fait que les particules en matériel inerte (20) sont des billes de verres d'un diamètre maximal de 3mm.

11. Appareil conforme à une des revendications 1 à 7 caractérisé par le fait que les particules en matériel inerte (20) sont des grains de sable.

12. Appareil conforme à une des revendications 1 à 7 caractérisé par le fait que les particules en matériel inerte (20) sont des grains de graphite.

13. Appareil conforme aux revendications 1 à 12 caractérisé par le fait qu' un dispositif de chauffage électrique (14) du tube métallique est équipé d'une sonde de température (22) mesurant la température des particules à l'intérieur du tube et permettant de régler la température des particules par un microprocesseur (4) de sorte à les chauffer à une valeur présélectionnée et la maintenir constante.

14. Appareil conforme à la revendication 13 caractérisé par le fait que son microprocesseur (4) électronique est équipé d'un dispositif pour présélectionner la température de travail et d'un indicateur de température lisible de l'extérieur.

15. Appareil conforme à une des revendications 1 à 14 caractérisé par le fait que le tube métallique (23) chauffant rempli de particules et le thermostat (17) de sécurité monté sur la plaque de base, le microprocesseur (4) pour le réglage et l'affichage de température lisible de l'extérieur, sont compactés dans un seul petit boîtier (1) commun qui a sur sa partie supérieure une ouverture circulaire (7) de façon à ce que les dimensions du boîtier avec ses fentes de refroidissement (2) et le tube d'isolation (16) entourant le tube métallique et la plaque d'isolation (19) séparant la plaque de base sont arrangés de telle manière que les parties électroniques et le boîtier ne dépassent jamais la température de 50°C, même si le tube chauffant atteint une température supérieure à 200°C.
